(19) Europäisches Patentamt
European Patent Office
Office européen des brevets

(11) Publication number: 0 194 840 B1

(12) **EUROPEAN PATENT SPECIFICATION**

(45) Date of publication of patent specification: **28.02.90**

(51) Int. Cl.$^5$: **C 07 D 307/935**

(21) Application number: **86301705.9**

(22) Date of filing: **10.03.86**

(54) **Process for producing an aldehydelactone.**

(30) Priority: **13.03.85 JP 50141/85**

(43) Date of publication of application:
**17.09.86 Bulletin 86/38**

(45) Publication of the grant of the patent:
**28.02.90 Bulletin 90/09**

(84) Designated Contracting States:
**CH DE FR GB LI**

(56) References cited:
**DE-A-2 523 695**
**CHEMICAL ABSTRACTS, vol. 96, no. 3, january 18, 1982, Columbus, Ohio, USA PAQUETTE, LEO A.; CROUSE, GARY D. "Stereo-controlled preparation of precursors to all primary prostaglandins from butadiene" page 411, column 1, abstract no. 19700g**
**CHEMICAL ABSTRACTS, vol. 82, no. 25, June 23, 1975Columbus, Ohio, USA KONDO, KIYOSI; MATSUMOTO, MASAKATSU; MORI, FUMIO "Bicyclicgamma-lactones from 2-cycloalkene-1,4- diols" page 471, column 1, abstract no. 170 163r**

(73) Proprietor: **Chisso Corporation**
**6-32, Nakanoshima 3-chome Kita-ku**
**Osaka-shi Osaka-fu (JP)**

(72) Inventor: **Kaneko, Chikara**
**11-401, Daiichi-Chiku Kawauchi Jutaku**
**Kawauchi Sendaishi Miyagiken (JP)**
Inventor: **Sato, Masayuki**
**12-23, Sakuragimachi**
**Sendaishi Miyagiken (JP)**

(74) Representative: **Ruffles, Graham Keith et al**
**MARKS & CLERK 57-60 Lincoln's Inn Fields**
**London WC2A 3LS (GB)**

Note: Within nine months from the publication of the mention of the grant of the European patent, any person may give notice to the European Patent Office of opposition to the European patent granted. Notice of opposition shall be filed in a written reasoned statement. It shall not be deemed to have been filed until the opposition fee has been paid. (Art. 99(1) European patent convention).

Courier Press, Leamington Spa, England.

## Description

This invention relates to a process for producing 3-oxo-6-formyl-2-oxabicyclo[3.3.0]-6-octene.

3-Oxo-6-formyl-2-oxabicyclo[3.3.0]-6-octene (hereinafter often referred to as "aldehydelactone") is a compound useful as an intermediate for producing prostaglandins. Prostaglandins are physiologically active substances having a variety of pharmacological activities, such as blood pressure depression, anti-ulteration, bronchodilation, inhibition of gastric acid secretion, partus induction, etc.

The known process for producing this aldehyelactone involve a large number of reaction steps. For example, L. A. Paquette *et al* reported a process for producing the aldehydelactone consisting of 12 to 14 reaction steps (see Tetrahedron, Vol. 37, page 281 (1981)). The process, however, is not an easy one for producing the intermediate, since it comprises a number of complicated reaction steps, and especially among these steps is included an ozonolysis reaction at the low temperature of −78°C.

The present inventors have made extensive research on a photo-addition reaction between a cis-2-cyclopenten-1,4-diol derivative and 2,2-dimethyl-1,3-dioxin-4-one, and have found that it is possible to obtain the aldehydelactone quickly without the need to isolate the photo-addition reaction products.

The object of the present invention is to provide a process for producing 3-oxo-6-formyl-2-oxabicyclo[3.3.0]-6-octene from raw materials which can be relatively easily prepared, the process having short steps consisting of easy unit reactions.

The present invention provides a process for producing 3-oxo-6-formyl-2-oxabicyclo[3.3.0]-6-octene which comprises effecting a photo-addition raction between 2,2-dimethyl-1,3-dioxin-4-one and *cis*-2-cyclopenten-1,4-diol, adding water, and heating the resulting mixture.

The production process of the present invention may be illustrated by the following reaction scheme:

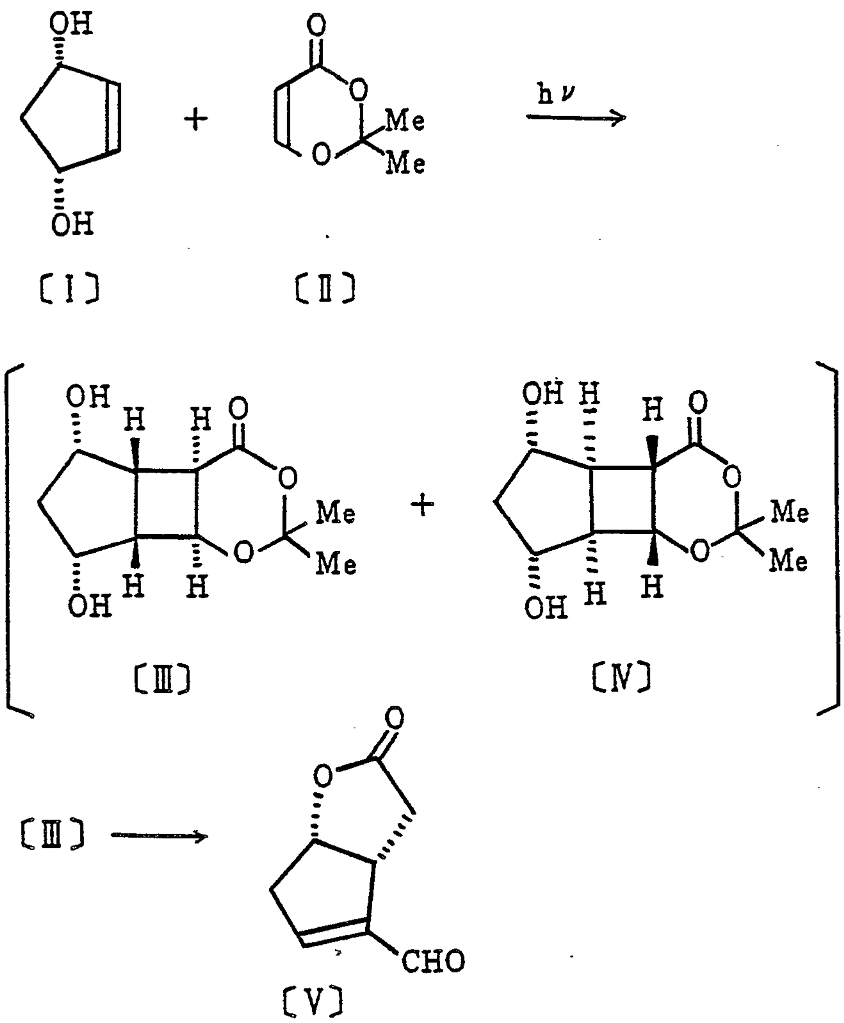


[I] [II] [III] [IV] [III] [V]

First, *cis*-2-cyclopenten-1,4-diol [I] (hereinafter often referred to as "diol") and 2,2-dimethyl-1,3-dioxin-4-one [II] (hereinafter often referred to as "dioxinone") are subjected to a photo-addition reaction typically in a suitable solvent, followed by adding water to the resulting adduct mixture and then heating the mixture

usually under reflux. In this way it is possible to obtain 3-oxo-6-formyl-2-oxabicyclo[3.3.0]-6-octene [V] via reaction steps of deacetonization, ring opening, lactonization, dehydration, etc.

For the proportion of the raw materials used for the addition reaction, the molar ratio of diol to dioxinone is usually 2:1 or more. In order to react dioxinone more effectively, the preferred molar ratio is in the range of 5:1 to 10:1. In other words, the diol is preferably used in large excess.

The nature of the solvent typically used in the present process has no particular limitation, provided that it is inert to the reaction. For example, esters such as ethyl acetate or butyl acetate; ethers such as tetrahydrofuran or dioxane; nitriles such as acetonitrile or propionitrile: or alcohols such as methanol or ethanol, may be used. In general the solvent will normally be a water-miscible solvent. The amount of solvent is preferably 1 to 100 l, more preferably, 10 to 50 l, per mole of dioxinone.

The photo-addition reaction is carried out preferably at a temperature of 25°C or lower, in order to inhibit side reactions, and more preferably in the range of 0°C to 20°C, although the reaction may be carried out even below 0°C, depending on the kind of the solvent, the irradiation conditions, etc.

The photo-addition proceeds by irradiation. As the light source for the irradiation, a high pressure or low pressure mercury vapour lamp is preferably used. As the irradiation light, the light at the ultraviolet portion within a wavelength range of 254 to 300 nm is preferred. Typical irradiation times for instance with a mercury vapour lamp are in the range of 0.1 to 3 hours, preferably 0.2 to 1 hour. The vessel for the photo-addition reaction is preferably not of a material having a high absorbency in the ultraviolet, in order to utilize the irradiation light effectively.

The addition reaction products comprise two kinds of isomers expressed by the above formulae (III) and (IV), and these isomers are formed in a nearly equal proportion: this can be presumed from the yields of the respective diacetate substances. Thus, the formation of the two isomers has been confirmed by converting the mixed adducts into their diacetates with acetic anhydride in the presence of pyridine, followed by chromatographic separation.

In the present invention, the adduct mixture is preferably freed from any solvent by distillation under reduced pressure or the like means.

Water is added to the adduct products and then the resulting mixture is heated, whereby the aldehydelactone is obtained from the isomer expressed by the formula [III]. The heating gives rise to reaction steps of hydrolysis, deacetonization, ring opening, lactonization, dehydration, etc. The amount of water is usually 2 to 400 moles, more preferably 50 to 250 moles, per mole of dioxinone.

The formylcarboxylic acid originating from the isomer of the formula (IV) is not lactonized, and is highly soluble in water so that it remains together with diol as an unreacted material in the aqueous layer. This solubility can be used to advantage when the desired aldehydelactone is extracted with a water-immiscible solvent. Hence there is an advantage that the objective product can be very easily separated. For extracting the objective aldehydelactone, halogenated hydrocarbons such as dichloromethane, ethylene chloride or chloroform, are preferably used.

As described above, the production process of the present invention basically comprises two unit steps of the addition reaction step and the lactonization step. The latter step can be carried out without separating the adducts from each other, and without purification. Hence the production process of the present invention can be regarded as substantially a single step production process.

*Cis*-2-cyclopenten-1,4-diol, one of the raw materials of the present process may be prepared for example by way of short steps including the photo-oxidation reaction of cyclopentadiene (Japanese patent application laid-open Sho 52-97940/1977). 2,2-Dimethyl-1,3-dioxin-4-one, the other raw material, may be prepared from Meldrum's acid as a commercially available reagent via formyl Meldrum's acid (Japanese patent application laid-open Sho 61-17580/1986).

According to the present process, the aldehydelactone can readily be produced with a yield of about 40% based on the raw material dioxinone. The objective 3-oxo-6-formyl-2-oxabicyclo[3.3.0]-6-octene could not previously be obtained without passing through a large number of steps, as described above. According to the present invention, a process for producing the compound using Meldrum's acid as a starting raw material, with a high overall yield of 15% or more has been established.

The present invention will be described in more detail by way of Example, but it is not limited thereto.

## Example

A solution of *cis*-2-cyclopenten-1,4-diol (2.50 g, 25 mols) and 2,2-dimethyl-1,3-dioxin-4-one (0.64 g, 5 mmols) dissolved in ethyl acetate (180 ml) was irradiated with a 400 W high pressure mercury vapour lamp using a Vycor glass filter, for 30 minutes to carry out a photo-addition reaction, followed by distilling off the solvent from the reaction mixture under reduced pressure to obtain an oily substance as residue (3.474 g). To this oily substance (1.388 g) was added distilled water (20 ml), followed by heating the mixture under reflux for 2 hours, thereafter cooling, three times carrying out extraction operations with dichloromethane (10 ml), combining the extracts, drying over anhydrous magnesium sulphate, and concentrating under reduced pressure to obtain an oily substance (275 mg), which was then subjected to silica gel column chromatography and eluted with diethyl ether to obtain a colourless syrupy product (123 mg; yield 40%).

This product had IR absorption spectra, $^1$H-NMR, MS and high resolution MS as shown below:

IR ($CHI_3$) ($cm^{-1}$): 1780 (O—C=O), 1680 (CH=O), 1620 (C=C).

$^1$H-NMR ($CDCl_3$) δ (ppm): 2.82 (2H, m, $CH_2CO$), 3.00 (2H, m, $CH_2$—C=C), 3.72 (1H, m, CH—C=C), 5.21 (1H, m, CHOC), 6.88 (1H, q, J=2Hz), 9.82 (1H, s, CHO).

MS m/z: 152 ($M^+$), 124, 95, 79, 67.

High resolution MS m/z: 152.0485.

(Calculated from $C_8H_8O_3$: 152.0473).

The results of IR, $^1$H-NMR and MS accorded well with the data reported by Tsung-Tee Li *et al* (see J. Org. Chem., Vol. 46, page 111 (1981)); thus the substance obtained above could be identified as 3-oxo-6-formyl-2-oxabicyclo[3.3.0]-6-actene.

## Claims

1. A process for producing 3-oxo-6-formyl-2-oxabicyclo[3.3.0]-6-octene which comprises effecting a photo-addition reaction between 2,2-dimethyl-1,3-dioxin-4-one and *cis*-2-cyclopenten-1,4-diol, adding water, and heating the resulting mixture.

2. A process according to claim 1, wherein the addition reaction is carried out at 25°C or lower, preferably from 0 to 20°C.

3. A process according to claim 1 or 2, wherein the addition reaction products are not isolated.

4. A process according to any preceding claim, wherein the 2,2-dimethyl-1,3-dioxin-4-one and *cis*-2-cyclopenten-1,4-diol are reacted in a molar ratio of at least 1:2 and preferably from 1:5 to 1:10.

5. A process according to any preceding claim, wherein the addition reaction is carried out in a solvent which is removed before the addition of water.

6. A process according to claim 5, wherein the solvent is a water-miscible solvent.

7. A process according to any preceding claim, wherein the desired 3-oxo-6-formyl-2-oxabicyclo[3.3.0]-6-octene is extracted with a water-immiscible solvent after the heating.

## Patentansprüche

1. Verfahren zur Herstellung von 3-oxo-6-formyl-2-oxabicyclo[3.3.0]-6-octene, dadurch gekennzeichnet, daß eine Lichtzugabereaktion zwischen 2,2-dimethyl-1,3-dioxin-4-on und cis-2-cyclopenten-1,4-diol durchgeführt wird, Wasser zugegeben wird und die sich ergebende Mischung erhitzt wird.

2. Verfahren nach Anspruch 1, dadurch gekennzeichnet, daß die Zugabereaktion bei 25°C oder weniger ausgeführt wird, vorzugsweise bei 0—20°C.

3. Verfahren nach Anspruch 1 oder 2, dadurch gekennzeichnet, daß die Produkte der Zugabereaktion nicht abgetrennt werden.

4. Verfahren nach einem der vorhergehenden Ansprüche, dadurch gekennzeichnet, daß das 2,2-dimethyl-1,3-dioxin-4-on und cis-2-cyclopenten-1,4-diol der Reaktion in einem molaren Verhältnis von wenigstens 1:2 und vorzugsweise von 1:5 bis 1:10 unterzogen werden.

5. Verfahren nach einem der vorhergehenden Ansprüche, dadurch gekennzeichnet, daß die Zugabereaktion in einem Lösungsmittel ausgeführt wird, das vor der Zugabe von Wasser entfernt wird.

6. Verfahren nach Anspruch 5, dadurch gekennzeichnet, daß das Lösungsmittel ein wassermischbares Lösungsmittel ist.

7. Verfahren nach einem der vorhergehenden Ansprüche, dadurch gekennzeichnet, daß das erwünschte 3-oxo-6-formyl-2-oxabicyclo-(3.3.0)-6-octene nach dem Erhitzen mit einem wasserunlöslichen Lösungsmittel extrahiert wird.

## Revendications

1. Procédé de préparation de "3-oxo-6-formyl-2-oxabicyclo-[3.3.0]-6-octene" consistant à effectuer une réaction de photo-addition entre la "2,2-diméthyl-1,3-dioxin-4-one" et le "cis-2-cyclopenten-1,4-diol", à ajouter de l'eau, et à chauffer le mélange résultant.

2. Procédé suivant la revendication 1, dans lequel la réaction d'addition est effectuée à 25°C ou à une température inférieure, de préférence de 0 à 20°C.

3. Procédé suivant la revendication 1 ou 2, dans lequel les produits de la réaction d'addition ne sont pas isolés.

4. Procédé suivant l'une quelconque des revendications précédentes, dans lequel la "2,2-diméthyl-1,3-dioxin-4-one" et le "cis-2-cyclopenten-1,4-diol" sont mis à réagir dans un rapport molaire d'au moins 1:2 et de préférence de 1:5 à 1:10.

5. Procédé suivant l'une quelconque des revendications précédentes, dans lequel la réaction d'addition est effectuée dans un solvant qui est éliminé avant l'ajout de l'eau.

6. Procédé suivant la revendication 5, dans lequel le solvant est un solvant miscible à l'eau.

7. Procédé suivant l'une quelconque des revendications précédentes, dans lequel le "3-oxo-6-formyl-2-oxabicyclo[3.3.0]-6-octene" souhaité est extrait à l'aide d'un solvant immiscible à l'eau, après le chauffage.